(19) Europäisches Patentamt
European Patent Office
Office européen des brevets

(11) Veröffentlichungsnummer : **0 042 451**
**B1**

(12) **EUROPÄISCHE PATENTSCHRIFT**

(45) Veröffentlichungstag der Patentschrift : **22.06.83**

(51) Int. Cl.³ : **C 12 P 33/08**, C 07 J 5/00, C 07 J 63/00// C07J21/00

(21) Anmeldenummer : **80103478.6**

(22) Anmeldetag : **21.06.80**

# (54) Verfahren zur Herstellung von 11-beta-Hydroxysteroiden.

(43) Veröffentlichungstag der Anmeldung : **30.12.81 Patentblatt 81/52**

(45) Bekanntmachung des Hinweises auf die Patenterteilung : **22.06.83 Patentblatt 83/25**

(84) Benannte Vertragsstaaten : **AT BE CH DE FR GB IT LI LU NL SE**

(56) Entgegenhaltungen :
**DE A 1 618 599**
**DE A 2 901 561**
**FR A 1 164 173**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Petzoldt, Karl, Dr.**
**Flachsweg 10**
**D-1000 Berlin 38 (DE)**
Erfinder : **Annen, Klaus, Dr.**
**Seegefelder Strasse 194**
**D-1000 Berlin 20 (DE)**
Erfinder : **Laurent, Henry, Dr.**
**Glambecker Weg 21**
**D-1000 Berlin 28 (DE)**

**EP 0 042 451 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von 11-beta-Hydroxysteroiden

Die Erfindung betrifft ein Verfahren zur Herstellung von 11β-Hydroxysteroiden der allgemeinen Formel I

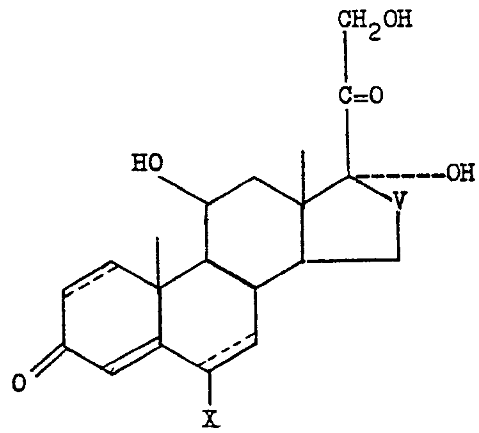

(I)

worin

die Bindungen ..... Einfachbindungen oder Doppelbindungen,

X ein Wasserstoffatom, ein Fluoratom, ein Chloratom oder eine Methylgruppe und

V eine Methylengruppe, eine Äthylengruppe, eine Äthylidengruppe oder eine Vinylidengruppe bedeuten, welches dadurch gekennzeichnet ist, daß man ein 11-Desoxysteroid der allgemeinen Formel II

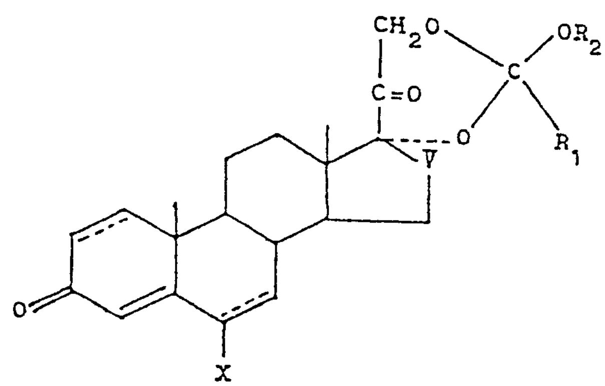

(II)

worin

..... X und V die obengenannte Bedeutung besitzen,

$R_1$ ein Wasserstoffatom oder eine 1 bis 6 Kohlenstoffatome enthaltende Alkylgruppe und

$R_2$ eine 1 bis 6 Kohlenstoffatome enthaltende Alkylgruppe bedeuten, mit einer Pilzkultur der Gattung Curvularia fermentiert.

Bekanntlich werden antiinflammatorisch wirksame 11β-Hydroxysteroide (wie zum Beispiel die Kortikoide : Hydrocortison, Prednisolon, Dexamethason, Betamethason, Prednyliden oder Flurandrenolon) mittels einer sehr aufwendigen vielstufigen Partialsynthese aus natürlich vorkommenden Steroiden (wie Diosgenin) hergestellt.

Innerhalb der vielstufigen Synthese dieser Verbindungen ist die mikrobiologische Einführung der 11β-Hydroxygruppe in das Steroidgerüst in der Regel der aufwendigste und wegen der dabei gebildeten Nebenprodukte, verlustreichste Syntheseschritt.

Im Jahre 1966 wurde ein Verfahren entwickelt, mit dessen Hilfe man die Ausbeute bei der 11β-Hydroxylierung von 11-Desoxy-17alpha-hydroxysteroiden der Pregnanreihe wesentlich steigern kann, indem man die 17alpha-Hydroxygruppe verestert, dann mittels Pilzen der Gattung Curvularia hydroxyliert und die erhaltenen 11β-Hydroxy-17alpha-acyloxysteroide verseift (Deutsches Patent 1 618 599).

Die für dieses bekannte Verfahren als Ausgangsverbindungen verwendeten 11-Desoxy-17alpha-acyloxysteroide können bekanntlich ihrerseits durch chemische Hydrolyse von 11-Desoxysteroiden der allgemeinen Formel II hergestellt werden.

Im Vergleich zu diesem vorbekannten dreistufigen Verfahren hat das erfindungsgemäße Verfahren den Vorzug, daß es als einstufiges Verfahren weniger aufwendig ist, und daß man mit seiner Hilfe höhere Ausbeuten an Verfahrensprodukt erzielt. Das erfindungsgemäße Verfahren hat darüberhinaus auch den

Vorteil, daß es überraschenderweise oft mit wesentlich höheren Substratkonzentrationen und mit kürzerer Reaktionszeit durchgeführt werden kann, als das bekannte Verfahren. Auch entfällt bei dem erfindungsgemäßen Verfahren die oft recht schwierige Hydrolyse der 11β-Hydroxy-17alpha-acyloxysteroide, bei der sich meist Nebenprodukte bilden, wodurch meist eine aufwendige und verlustreiche Aufreinigung der erhaltenen Produkte erforderlich ist, damit diese den für Arzneimittelwirkstoffen erforderlichen Reinheitskriterien entsprechen.

Ferner ist bemerkenswert, daß man nach dem erfindungsgemäßen Verfahren auch sehr gut 11β-Hydroxy-delta $^{1,4}$-steroide der allgemeinen Formel I aus den entsprechenden 11-Desoxy-delta$^{1,4}$-steroiden der allgemeinen Formel II herstellen kann, während es nach dem bekannten Stand der Technik nicht möglich ist 11-Desoxy-delta$^{1,4}$-steroide in guten Ausbeuten in der 11β-Position zu hydroxylieren.

Letztlich ist erwähnenswert, daß es für die Durchführung des erfindungsgemäßen Verfahrens nicht erforderlich ist asl Ausgangsverbindungen Reinprodukte der 11-Desoxysteroide der allgemeinen Formel II zu verwenden, wenn man diese beispielsweise nach folgender Methode als Rohprodukt herstellt :

Aus einer Lösung von 1 g (4 mmol) Pyridiniumtosylat und 10 g (28 mmol) 17alpha, 21-Dihydroxysteroid in 80 ml Dimethylformamid und 700 ml Benzol werden zur Entfernung von Wasserspuren bei einer Badtemperatur von 110-130 °C (Glycerinbad) 300 ml Benzol unter Anwendung eines Wasserabscheiders abdestilliert. Anschließend kühlt man kurz ab, fügt 24 ml (110-140 mmol) Orthocarbonsäuretrialkylester hinzu und destilliert innerhalb von 1,5 Stunden das restliche Benzol ab. Nach Zugabe von 5,2 ml Pyridin wird das Reaktionsprodukt nunmehr im Hochvakuum weiter zur Trockne eingeengt.

Die Reaktion verläuft quantitativ. Das nach dem Einengen zurückbleibende 17alpha, 21-(1-Alkoxyalkylidendioxy)-steroid ist zunächst von öliger Konsistenz, kristallisiert jedoch meist nach kurzer Zeit durch. Der Reinheitsgrad ist für die sich anschließende mikrobiologische Hydroxylierung völlig ausreichend, die dem Kristallisat noch anhaftenden Reste der verwendeten Reagenzien stören die Enzymkatalyse nicht. Analog werden die D-Homo-steroide der allgemeinen Formel II hergestellt.

(Das Pyridiniumtosylat wird folgendermaßen hergestellt : 54 g p-Toluolsulfonsäure mal $H_2O$ werden zur Entfernung des Wassers dreimal mit einer ausreichenden Menge Benzol im Vakuum zur Trockne eingeengt. Den Rückstand versetzt man mit 240 ml Pyridin, rührt 1/2 Stunde nach und fällt das Produkt mit Äther aus. Das Kristallisat wird abgesaugt, mit Äther gewaschen und 1 Stunde bei 50 °C im Vakuum getrocknet.)

Die 11-Desoxysteroide der allgemeinen Formel II können als Alkylgruppen $R_1$ und $R_2$ verzweigtkettige oder vorzugsweise geradkettige Gruppen mit 1 bis 6 Kohlenstoffatomen besitzen. Geeignete Alkylgruppen $R_1$ und $R_2$ sind beispielsweise die Methylgruppe, die Äthylgruppe, die Propylgruppe, die Isopropylgruppe, die Butylgruppe, die Pentylgruppe und die Hexylgruppe. Eine besonders bevorzugte Alkylgruppe $R_1$ ist die Methylgruppe. Besonders bevorzugte Alkylgruppen $R_2$ sind die Methylgruppe und die Äthylgruppe.

Bei den in der 6,7-Position gesättigten 11-Desoxysteroiden der allgemeinen Formel I ist der Substituent X vorzugsweise alpha-ständig.

Daß sich die 11-Desoxysteroide der allgemeinen Formel II hydroxylieren lassen und zu den entsprechenden 11β-Hydroxysteroiden der allgemeinen Formel I gespalten werden, ist für den Fachmann überraschend. Noch weniger war vorhersehbar, daß bei dem erfindungsgemäßen Verfahren die erwünschten Verfahrensprodukte in sehr hohen Ausbeuten (von etwa 85-90 % der Theorie) erhalten werden.

Abgesehen von der Verwendung anderer Ausgangsverbindungen wird das erfindungsgemäße Verfahren unter den Bedingungen durchgeführt, die man üblicherweise zur 11β-Hydroxylierung von Steroiden mit Pilzen der Gattung Curvularia anwendet.

Zur Hydroxylierung geeignete Pilze der Gattung Curvularia sind beispielsweise Curvularia falcuta QM-102 H, Curvularia genticulata IFO (6284), Curvularia lunata NRRL 2380, NRRL 2434, NRRL 2178, ATCC 12017 oder IFO (6286) oder Curvularia maculans IFO (6292).

Unter den für diese Mikroorganismen üblicherweise verwendeten Kulturbedingungen werden in einem geeigneten Nährmedium unter Belüften Submerskulturen angezüchtet. Dann setzt man den Kulturen das Substrat (in einem geeigneten Lösungsmittel gelöst oder vorzugsweise in emulgierter Form) zu und fermentiert, bis eine maximale Substratumwandlung erreicht ist.

Geeignete Substratlösungsmittel sind beispielsweise Methanol, Äthanol, Glykolmonomethyläther, Dimethylformamid oder Dimethylsulfoxyd. Die Emulgierung des Substrats kann beispielsweise bewirkt werden, in dem man dieses in mikronisierter Form oder in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxydaddukte oder Fettsäureester von Polyglykolen. Als geeignete Emulgatoren seien die handelsüblichen Netzmittel Tegin$^{(R)}$, Tagat$^{(R)}$, Tween$^{(R)}$ und Span$^{(R)}$ beispielsmäßig genannt.

Oft ermöglicht die Emulgierung der Substrate einen erhöhten Substratdurchsatz und somit eine Steigerung der Substratkonzentration. Es ist aber selbstverständlich auch möglich, bei dem erfindungsgemäßen Verfahren andere Methoden zur Steigerung des Substratdurchsatzes, wie sie dem Fermentationsfachmann wohl bekannt sind, anzuwenden.

Die optimale Substratkonzentration, Substratzugabezeit und Fermentationsdauer ist von der Struktur des verwendeten Substrates und der Art des verwendeten Mikroorganismus abhängig. Diese Größen müssen, wie dies bei mikrobiologischen Steroidumwandlungen allgemein erforderlich ist, im Einzelfall durch Vorversuche, wie sie dem Fachmann geläufig sind, ermittel werden.

Nach dem erfindungsgemäßen Verfahren lassen sich aus den entsprechenden 11-Desoxysteroiden der allgemeinen Formel II beispielsweise folgende 11β, 17alpha, 21-Trihydroxy-4-pregnen-3,20-dion-Derivate der allgemeinen Formel I herstellen :

11β,17alpha,21-Trihydroxy-4-pregnen-3,20-dion,
11β,17alpha,21-Trihydroxy-1,4-pregnadien-3,20-dion,
11β,17alpha,21-Trihydroxy-6alpha-methyl-4-pregnen-3,20-dion,
11β,17alpha,21-Trihydroxy-6-alpha-methyl-1,4-pregnadien-3,20-dion,
11β,17alpha,21-Trihydroxy-6-chlor-4,6-pregnadien-3,20-dion,
11β,17alpha,21-Trihydroxy-6-chlor-1,4,6-pregnatrien-3,20-dion,
11β,17alpha,21-Trihydroxy-16alpha-methyl-4-pregnen-3,20-dion,
11β,17alpha,21-Trihydroxy-16alpha-methyl-1,4-pregnadien-3,20-dion,
11β,17alpha,21-Trihydroxy-16β-methyl-4-pregnen-3,20-dion,
11β,17alpha,21-Trihydroxy-16β-methyl-1,4-pregnadien-3,20-dion,
11β,17alpha,21-Trihydroxy-16-methylen-4-pregnen-3,20-dion,
11β,17alpha,21-Trihydroxy-16-methylen-1,4-pregnadien-3,20-dion,
11β,17alpha,21-Trihydroxy-D-homo-4-pregnen-3,20-dion,
11β,17alpha,21-Trihydroxy-D-homo-1,4-pregnadien-3,20-dion,
6alpha-Fluor-11β,17alpha,21-trihydroxy-4-pregnen-3,20-dion und
6alpha-Fluor-11β,17alpha,21-trihydroxy-1,4-pregnadien-3,20-dion.

Die nachfolgenden Ausführungsbeispiele dienen zur Erläuterung des erfindungsgemäßen Verfahrens :

## Beispiel 1

a) 8 g Pyridiniumtosylat und 80 g 17alpha, 21-Dihydroxy-4-pregnen-3,20-dion werden in 560 ml Dimethylformamid gelöst und mit 3,5 l Benzol verdünnt. Danach destilliert man bei einer Badtemperatur von 120 °C über einen Wasserabscheider 1,5 l Benzol ab. In die heiße Reaktionslösung läßt man langsam 192 ml Orthoessigsäuretriethylester zulaufen und destilliert anschließend 1 Stunde lang Benzol und andere leicht flüchtige Reaktionskomponenten ab. Man fügt nun 96 ml Pyridin hinzu und engt bei einer Badtemperatur von 60 °C im Hochvakuum im Verlauf von 2 Stunden vollständig zur Trockne ine. Der zunächst ölige Rückstand kristallisiert schnell durch. Man erhält 113 g 17alpha,21-(1-Ethoxy-ethylidendioxy)-4-pregnen-3,20-dion in Form gelber Kristalle vom Schmelzpunkt 89-90 °C.

b) Ein 2 l-Erlenmeyerkolben, der 500 ml einer 30 Minuten bei 120 °C im Autoklaven sterilisierten Nährlösung aus 3 % Glukose, 1 % Corn steep, 0,2 % $NaNO_3$, 0,1 % $KH_2PO_4$, 0,2 % $K_2HPO_4$, 0,05 % $MgSO_4 \cdot 7H_2O$, 0,002 %, $FeSO_4 \cdot 7H_2O$ und 0,05 % KCl enthält, wird mit einer Lyophilkultur von Curvularia lunata (NRRL 2380) beimpft und 60 Stunden bei 30 °C auf einem Rotationsschüttler bewegt. Diese Anzuchtskultur (250 ml) dient zur Beimpfung eines 20 l-Vorfermenters, der mit 15 l eines bei 121 °C und 1,1 atü sterilisierten Nährmediums, bestehend aus 2 % Glukose und 2 % Corn steep, eingestellt auf pH 6,5, beschickt ist. Unter Zugabe von Silicon SH als Antischaummittel wird nun bei 29 °C und 0,7 atü Druck unter Belüftung (10 l/Min.) und Rühren (220 U/Min.) 24 Stunden germiniert. Danach werden 1,5 l dieser Kultur unter sterilen Bedingungen entnommen und damit ein 20 l-Hauptfermenter beimpft, der mit 14 l eines wie oben sterilisierten Nährmediums aus 3 % Corn steep und 0,7 % Glukose, eingestellt auf pH 6,5 gefüllt ist.

Nach einer Anwachsphase von 12 Stunden unter Vorfermenterbedingungen werden, 7,5 g rohes 17alpha,21-(1-Ethoxy-ethyl-idendioxy)-4-pregnen-3,20-dion, die 6,43 g Reinsubstanz enthalten, gelöst in 100 ml Dimethylformamid, unter sterilen Bedingungen hinzugegeben und weiter gerührt und belüftet. Der Verlauf der Fermentation wird durch Entnahme von Proben kontrolliert, die mittels Methylisobutylketon extrahiert und dünnschichtchromatographiert analysiert werden. Nach 38 Stunden Kontaktzeit ist die Umsetzung des Substrates beendet.

Der Fermenterinhalt wird filtriert, das Kulturfiltrat als auch das abfiltrierte Mycel mit Methylisobutylketon extrahiert, die Extrakte vereinigt und zunächst im Umlaufverdampfer konzentriert, anschließend bei 50 °C Badtemperatur im Vakuum im Rotationsverdampfer zur Trockne eingeengt. Den Rückstand löst man in warmen Methanol, filtriert vom ungelöst gebliebenen Siliconöl ab und dampft wieder zur Trockne ein. Der verbliebene Rückstand (9,4 g) wird in 40 ml Essigsäureethylester in der Wärme gelöst und zunächst bei Raumtemperatur, anschließend in der Tiefkühltruhe kristallisiert. Das auskristallisierte Produkt wird abgesaugt, mit wenig kaltem Essigester gawaschen und 5 Stunden bei 60 °C im Vakuumtrockenschrank getrocknet. Man erhält 4,18 g Hydrocortison (11β,17alpha-,21-Trihydroxy-4-pregnen-3,20-dion) vom Schmelzpunkt 212-214 °C. Die Kristallisat-Mutterlauge wird mit Aktivkohle erwärmt, filtriert und auf 15 ml Volumen konzentriert. Beim Abkühlen und Stehen über Nacht bei

Raumtemperatur kristallisieren weitere 0,7 g Hydrocortison vom Schmelzpunkt 208-210 °C aus, so daß die Gesamtausbeute 87,1 % der Theorie beträgt.

Beispiel 2

a) 7 g Pyridiniumtosylat und 70 g 17alpha,21-Dihydroxy-4-pregnen-3,20-dion werden in 560 ml Dimethylformamid gelöst und mit 4,9 l Benzol verdünnt. Man destilliert bei einer Badtemperatur von 130 °C, 2,1 l Benzol über einen Wasserabscheider ab und fügt nach kurzem Abkühlen 168 ml Orthobuttersäuretriethylester hinzu. Innerhalb von 1,5 Stunden destilliert man bei 130 °C das restliche Benzol ab. Die Lösung wird mit 84 ml Pyridin versetzt und anschließend am Rotationsverdampfer im Hochvakuum zur Trockne eingeengt. Man erhält 103,8 g 17alpha,21-(1-Ethoxy-butyl-idendioxy)-4-pregnen-3,20-dion als Öl.

b) Unter den Bedingungen des Beispiels 1b werden 7,5 g rohes 17alpha,21-(1-Ethoxy-butylidendioxy)-4-pregnen-3,20-dion, die 6,3 g Reinsubstanz enthalten, mit einer Curvularia lunata-Kultur 47 Stunden fermentiert. Nach der Aufarbeitung des Ansatzes und Kristallisation des vom Silicon befreiten Extraktrückstandes aus Essigsäureethylester erhält man insgesamt 4,3 g Hydrocortison vom Schmelzpunkt 210-213 °C.

Beispiel 3

a) 5,3 g Pyridiniumtosylat und 53 g 17alpha,21-Dihydroxy-6alpha-methyl-4-pregnen-3,20-dion werden in 370 ml Dimethylformamid gelöst und mit 2,5 l Benzol verdünnt. Danach destilliert man bei einer Badtemperatur von 130 °C über einen Wasserabscheider 1 l Benzol ab. In die heiße Reaktionslösung läßt man 127 l Orthoessigsäureethylester langsam zulaufen und destilliert anschließend 1 Stunde lang Benzol und andere leicht flüchtige Reaktionskomponenten ab. Man fügt nun 63 ml Pyridin hinzu und engt bei einer Badtemperatur von 60 °C im Hochvakuum im Verlauf von 2 Stunden vollständig zur Trockne ein. Es hinterbleiben 66,8 g öliges 17alpha,21-(1-Ethoxy-ethylidendioxy)-6alpha-methyl-4-pregnen-3,20-dion.

Eine Probe des öligen Rückstandes wird mit dest. Wasser intensiv gerührt, wobei das Öl langsam in den kristallinen Zustand übergeht. Schmelzpunkt 75/92-95 °C.

b) Unter den Bedingungen des Beispiels 1b werden 6 g rohes 17alpha,21-(1-Ethoxy-ethylidendioxy)-6alpha-methyl-4-pregnen-3,20-dion, die 4,8 g Reinsubstanz enthalten, mit einer Curvularia lunata — Kultur 51 Stunden fermentiert. Nach der Aufarbeitung des Ansatzes und Kristallisation des Extraktrückstandes aus Diisopropyläther erhält man 3,4 g 6alpha-Methyl-hydrocortison vom Schmelzpunkt 217-219 °C.

Beispiel 4

a) 4 g Pyridiniumtosylat und 40 g 17alpha,21-Dihydroxy-1,4-pregnadien-3,20-dion werden in 280 ml Dimethylformamid gelöst und mit 2 l Benzol verdünnt. Danach destilliert man bei einer Badtemperatur von 120 °C über einen Wasserabscheider 600 ml Benzol ab. In die heiße Reaktionslösung läßt man 96 ml Orthoessigsäuretriethylester langsam zulaufen und destilliert anschließend weiter Benzol und andere leicht flüchtige Reaktionskomponenten ab. Man fügt nun 48 ml Pyridin hinzu und engt bei einer Badtemperatur von 60 °C im Hochvakuum im Verlauf von 2 Stunden vollständig zur Trockne ein. Man erhält 58 g 17alpha,21-(1-Ethoxy-ethylidendioxy)-1,4-pregnadien-3,20-dion als gelbliches Kristallisat.

Eine Probe kristallisiert man nach Behandlung mit A-Kohle aus Äther mit 1 % Aceton um und erhält ein Reinprodukt vom Schmelzpunkt 153-154 °C.

b) Ein 2 l-Erlenmeyerkolben, der 500 ml einer 30 Min. bei 120 °C im Autoklaven sterilisierten Nährlösung aus 3 % Glukose, 1 % Corn steep, 0,2 % $NaNO_3$, 0,1 % $KH_2PO_4$, 0,2 % $K_2HPO_4$, 0,05 $MgSO_4 \cdot 7H_2O$, 0,002 % $FeSO_4 \cdot 7H_2O$ und 0,05 % KCl enthält, wird mit einer Lyophilkultur von Curvularia lunata (NRRL 2380) beimpft und 60 Stunden bei 30 °C auf einem Rotationsschüttler bewegt. Diese Anzuchtskultur (250 ml) dient zur Beimpfung eines 20 l-Vorfermenters, der mit 15 l eines bei 121 °C und 1,1 atü sterilisierten Nährmediums, bestehend aus 2 % Glukose und 2 % Corn steep, eingestellt auf pH 6,5 beschickt ist. Unter Zugabe von Silicon SH als Antischaummittel wird bei 29 °C und 0,7 atü Druck unter Belüftung (10 l/Min.) und Rühren (120 U/Min.) 24 Stunden germiniert. Danach werden 1,5 l dieser Kultur unter sterilen Bedingungen entnommen und damit ein 20 l-Hauptfermenter beimpft, der mit 14 l eines wie oben sterilisierten Nährmediums aus 3 % Corn steep und 0,7 % Glukose, eingestellt auf pH 5,5 gefüllt ist.

Nach einer Anwachsphase von 12 Stunden unter Vorfermenterbedingungen werden 7,5 g 17alpha, 21-(1-Ethoxy-ethylidendioxy)-1,4-pregandien-3,20-dion, gelöst in 100 ml Dimethylformamid, unter sterilen Bedingungen hinzugegeben und weiter gerührt und belüftet. Der Verlauf der Fermentation wird durch Entnahme von Proben kontrolliert, die mittles Methylisobutylketon extrahiert und dünnschichtchromatographisch analysiert werden. Nach 36 Stunden Kontaktzeit ist die Umsetzung des Substrates beendet. Der Fermenterinhalt wird filtriert, das Kulturfiltrat als auch das abfiltrierte Mycel mit Methylisobutylketon extrahiert, die Extrakte vereinigt und zunächst im Umlaufverdampfer konzentriert, anschließend bei 50 °C Badtemperatur im Vakuum im Rotationsverdampfer auf 100 ml eingeengt und bei Raumtemperatur

kristallisieren lassen. Das Produkt wird abgesaugt, im Vakuum getrocknet und man erhält 5,7 g Prednisolon vom Schmelzpunkt 229-231 °C.

Beispiel 5

a) 0,62 g Pyridiniumtosylat und 6,2 g D-Homo-Reichstein S werden in 43,4 ml Dimethylformamid gelöst und mit 270 ml Benzol verdünnt. Danach destilliert man bei einer Badtemperatur von 120 °C über einen Wasserabscheider 116 ml Benzol ab. In die heiße Reaktionslösung läßt man langsam 14,9 ml Orthoessigsäuretrimethylester zulaufen und destilliert anschließend 1 Stunde lang Benzol und andere leicht flüchtige Reaktionskomponenten ab. Man fügt nun 7,5 ml Pyridin hinzu und engt bei einer Badtemperatur von 60 °C im Hochvakuum im Verlauf von 2 Stunden vollständig zur Trockne ein. Der ölige Rückstand wird aus Ether kristallisiert. Man erhält 7,3 g 17,21-(1-Methoxy-ethylidendioxy)-D-homo-4-pregnen-3,20-dion in Form gelber Kristalle vom Schmelzpunkt 167-169 °C.

b) Ein 2 l-Erlenmeyerkolben, der 500 ml einer 30 Min. bei 120 °C im Autoklaven sterilisierten Nährlösung aus 3 % Glukose, 1 % Corn steep, 0,02 % $NaNO_3$, 0,1 % $KH_2PO_4$, 0,2 % $K_2HPO_4$, 0,05 % $MgSO_4 \cdot 7H_2O$, 0,002 % $FeSO_4 \cdot 7H_2O$ und 0 05 % KCl enthält, wird mit einer Lyophilkultur von Curvularia lunata (NRRL 2380) beimpft und 60 Stunden bei 30 °C auf einem Rotationsschüttler bewegt. Diese Anzuchtskultur (250 ml) dient zur Beimpfung eines 20 l-Vorfermenters, der mit 15 l eines bei 121 °C und 1,1 atü sterilisierten Nährmediums, bestehend aus 2 % Glukose und 2 % Corn steep, eingestellt auf pH 6,5, beschickt ist. Unter Zugabe von Silicon SH als Antischaummittel wird nun bei 29 °C und 0,7 atü Druck unter Belüftung (10 l/Min.) und Rühren (220 U/Min.) 24 Stunden germiniert. Danach werden 1,5 l dieser Kultur unter sterilen Bedingungen entnommen und damit ein 20 l-Hauptfermenter beimpft, der mit 14 l eines wie oben sterilisierten Nährmediums aus 3 % Corn steep und 0,7 % Glukose, eingestellt auf pH 6,5, gefüllt ist. Nach einer Anwachsphase von 12 Stunden unter Vorfermenterbedingungen werden 7,3 g 17, 21-(1-Methoxy-ethylidendioxy)-D-homo-4-pregnen-3,20-dion, gelöst in 100 ml Dimethylformamid, unter sterilen Bedingungen hinzugegeben und weiter gerührt und belüftet. Der Verlauf der Fermentation wird durch Entnahme von Proben kontrolliert, die mittels Methylisobutylketon extrahiert und dünnschichtchromatographiert analysiert werden. Nach 48 Stunden Kontaktzeit ist die Umsetzung des Substrates beendet.

Der Fermenterinhalt wird filtriert, das Kulturfiltrat als auch das abfiltrierte Mycel mit Methylisobutylketon extrahiert, die Extrakte vereinigt und zunächst im Umlaufverdampfer konzentriert, anschließend bei 50 °C Badtemperatur im Vakuum im Rotationsverdampfer zur Trockne eingeengt. Den Rückstand löst man in warmen Methanol, filtriert vom ungelöst gebliebenen Siliconöl ab und dampft wieder zur Trockne ein. Der verbliebene Rückstand wird über eine Kieselgelsäule (Gradient 4,5 l Methylenchlorid/0,5 l Aceton-4 l Methylenchlorid/1 l Aceton) chromatographiert und aus Aceton kristallisiert. Man erhält 5,7 g D-Homo-Hydrocortison (11β-17alpha,21-Trihydroxy-D-homo-4-pregnen-3,20-dion) vom Schmelzpunkt 212-215 °C (Zers.).

Beispiel 6

a) 1,5 g Pyridiniumtosylat und 15 g 17alpha,21-Dihydroxy-D-homo-1,4-pregnadien-3,20-dion werden in 120 ml Dimethylformamid gelöst und mit 800 ml Benzol verdünnt. Danach destilliert man bei einer Badtemperatur von 120 °C über einen Wasserabscheider 250 ml Benzol ab. In die heiße Reaktionslösung läßt man 40 ml Orthoessigsäuretrimethylester langsam zulaufen und destilliert anschließend weiter Benzol und andere leicht flüchtige Reaktionskomponenten ab. Man fügt nun 20 ml Pyridin hinzu engt bei einer Badtemperatur von 60 °C im Hochvakuum im Verlauf von 2 Stunden vollständig zur Trockne ein. Es hinterbleiben 18,8 g ölig-kristallines Rohprodukt. Eine Probe kristallisiert man nach Behandlung mit A-Kohle aus Isopropylether um und erhält reines 17alpha,21-(1-Methoxy-ethylidendioxy)-D-homo-1,4-pregnadien-3,20-dion vom Schmelzpunkt 142-144 °C.

b) Ein 2 l-Erlenmeyerkolben, der 500 ml einer 30 Min . bei 120 °C im Autoklaven sterilisierten Nährlösung aus 3 % Glukose, 1 % Corn steep, 0,2 % $NaNO_3$, 0,1 % $KH_2PO_4$, 0,2 % $K_2HPO_4$, 0,05 % $MgSO_4 \cdot 7H_2O$, 0,002 % $FeSO_4 \cdot 7H_2O$ und 0,05 % KCl enthält, wird mit einer Lyophilkultur von Curvularia lunata (NRRL 2380) beimpft und 60 Stunden bei 30 °C auf einem Rotationsschüttler bewegt. Diese Anzuchtskultur (250 ml) dient zur Beimpfung eines 20 l-Vorfermenters, der mit 15 l eines bei 121 °C und 1,1 atü sterilisierten Nährmediums, bestehend aus 2 % Glukose und 2 % Corn steep, eingestellt auf pH 6,5, beschickt ist. Unter Zugabe von Silicon SH als Antischaummittel wird bei 29 °C und 0,7 atü Druck unter Belüftung (10 l/Min.) und Rühren (220 U/Min.) 24 Stunden germiniert. Danach werden 1,5 l dieser Kultur unter sterilen Bedingungen entnommen und damit ein 20 l-Hauptfermenter beimpft, der mit 14 l eines wie oben sterilisierten Nährmediums aus 3 % Corn steep und 0,7 % Glukose, eingestellt auf pH 5,5, gefüllt ist.

Nach einer Anwachsphase von 12 Stunden unter Vorfermenter-bedingungen werden 7,5 g 17alpha, 21-(1-Methoxy-ethylidendioxy)-D-homo-1,4-pregnadien-3,20-dion, gelöst in 100 ml Dimethylformamid, unter sterilen Bedingungen hinzugegeben und weiter gerührt und belüftet. Der Verlauf der Fermentation wird durch Entnahme von Proben kontrolliert, die mittels Methylisobutylketon extrahiert und dünnschichtchromatographisch analysiert werden. Nach 52 Stunden Kontaktzeit ist die Umsetzung des Substrates beeendet. Der Fermenterinhalt wird filtriert, das Kulturfiltrat als auch das abfiltrierte Mycel mit

Methylisobutylketon extrahiert, die Extrakte vereinigt und zunächst im Umlauf-verdampfer konzentriert, anschließend bei 50 °C Badtemperatur im Vakuum im Rotationsverdampfer auf 100 ml eingeengt und bei Raumtemperatur kristallisieren lassen. Das Produkt wird abgesaugt und im Vakuum getrocknet. Man erhält 6,1 g D-Homo-Prednisolon (11β,17alpha,21-Trihydroxy-D-homo-1,4-pregnadien-3,20-dion vom Schmelzpunkt 226-228 °C.

**Ansprüche**

1. Verfahren zur Herstellung von 11β-Hydroxysteroiden der allgemeinen Formel I

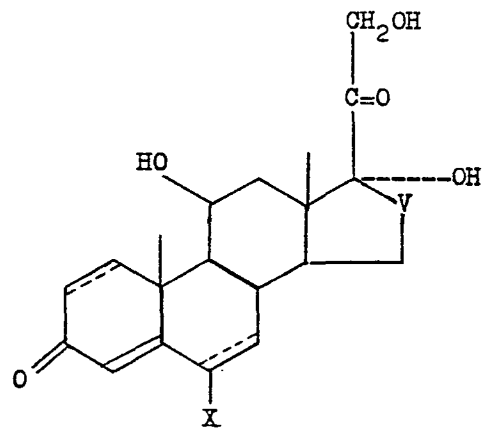

(I)

worin

die Bindungen ..... Einfachbindungen oder Doppelbindungen,

X ein Wasserstoffatom, ein Fluoratom, ein Chloratom oder eine Methylgruppe und

V eine Methylengruppe, eine Äthylengruppe, eine Äthylidengruppe oder eine Vinylidengruppe bedeuten, dadurch gekennzeichnet, daß man ein 11-Desoxysteroid der allgemeinen Formel II

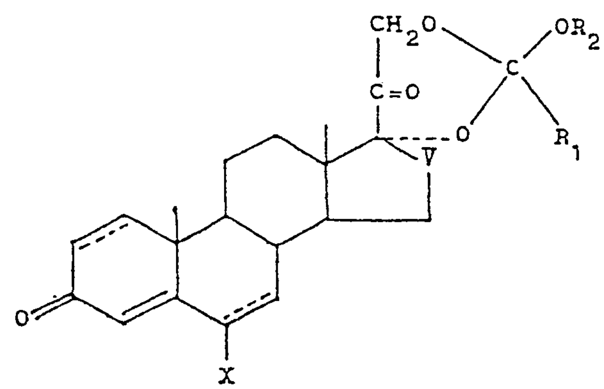

(II)

worin

......, X und V die obengenannte Bedeutung besitzen,

$R_1$ ein Wasserstoffatom oder eine 1 bis 6 Kohlenstoffatome enthaltende Alkylgruppe und

$R_2$ eine 1 bis 6 Kohlenstoffatome enthaltende Alkylgruppe bedeuten, mit einer Pilzkultur der Gattung Curvularia fermentiert.

2. Verfahren zur Herstellung von 11β-Hydroxysteroiden der allgemeinen Formel I, gemäß Anspruch 1, dadurch gekennzeichnet, daß man die Fermentation mit Hilfe einer Pilzkultur der Species Curvularia lunata durchführt.

3. Verfahren zur Herstellung von 11β-Hydroxy-D-homo-steroiden der allgemeinen Formel I gemäß Anspruch 1 und 2.

**Claims**

1. Process for the manufacture of 11β-hydroxysteroids of the general formula I

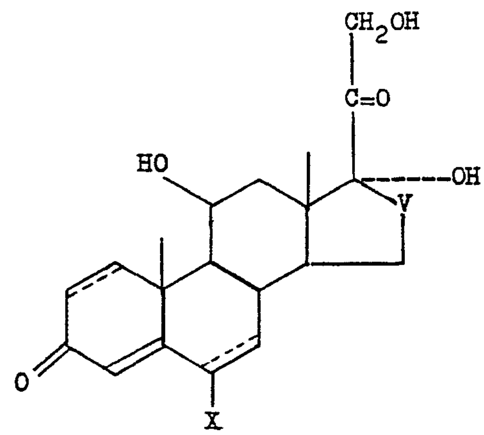

(I)

in which

the bonds ..... represent single bonds or double bonds,

X represents a hydrogen atom, a fluorine atom, a chlorine atom or a methyl group, and

V represents a methylene group, an ethylene group, an ethylidene group or a vinylidene group, characterised in that a 11-desoxysteroid of the general formula II

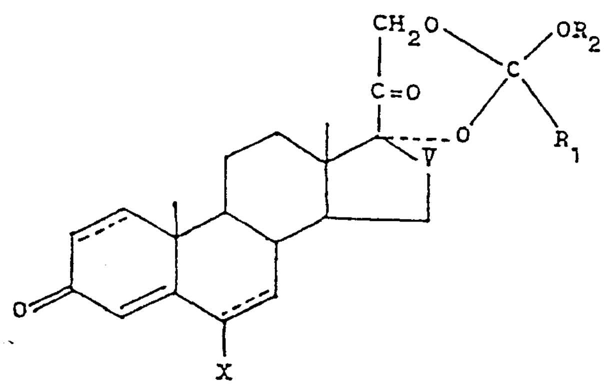

(II)

in which

....., X and V have the meanings given above,

$R_1$ represents a hydrogen atom or an alkyl group containing from 1 to 6 carbon atoms, and

$R_2$ represents an alkyl group containing from 1 to 6 carbon atoms, is fermented with a fungal culture of the genus Curvularia.

2. Process for the manufacture of 11β-hydroxysteroids of the general formula I according to claim 1, characterised in that fermentation is carried out with a fungal culture of the species Curvularia lunata.

3. Process for the manufacture of 11β-hydroxy-D-homosteroids of the general formula I according to claims 1 and 2.

**Revendications**

1. Procédé de préparation d'hydroxy-11β stéroïdes répondant à la formule générale (I)

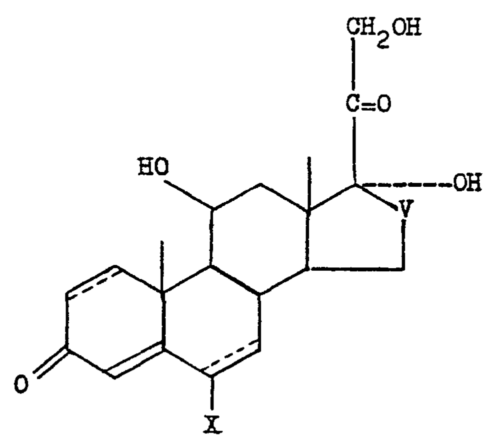

(I)

dans laquelle

les liaisons ..... sont des liaisons simples ou des liaisons doubles,

X représente un atome d'hydrogène, un atome de fluor, un atome de chlore ou un radical méthyle, et

V représente un radical méthylène, éthylène, éthylidène ou vinylidène, procédé caractérisé en ce qu'on fait fermenter au moyen d'une culture de mycètes du genre Curvularia un désoxy-11 stéroïde répondant à la formule générale (II)

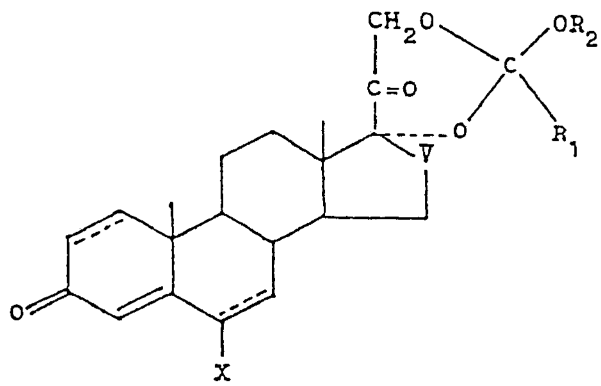

(II)

dans laquelle

......, X et V ont les significations indiquées ci-dessus,

$R_1$ représente un atome d'hydrogène ou un radical alkyle contenant de 1 à 6 atomes de carbone, et

$R_2$ représente un radical alkyle contenant de 1 à 6 atomes de carbone.

2. Procédé de préparation d'hydroxy-11 stéroïdes de formule générale (I) selon la revendication 1, procédé caractérisé en ce qu'on effectue la fermentation au moyen d'une culture de mycètes de l'espèce Curvularia lunata.

3. Procédé de préparation d'hydroxy-11 D-homo-stéroïdes de formule générale (I) selon l'une des revendications 1 et 2.